# EUROPÄISCHE PATENTANMELDUNG

(11) **EP 1 637 585 A1**
(43) Veröffentlichungstag der Anmeldung: **22.03.2006**
(21) Anmeldenummer: 05019984.3
(22) Anmeldetag: 14.09.2005
(51) Int. Cl.: C12M 1/107

(54) **Verfahren zur Erhöhung der Gasausbeute einer Biogasanlage**

(30) Priorität: 17.09.2004 DE 102004045239
(71) Anmelder: Gürtner, Michael, 86558 Hohenwart (DE)
(72) Erfinder: Gürtner, Michael, 86558 Hohenwart (DE)
(74) Vertreter: Seidel, Herta

(57) **Zusammenfassung**

Verfahren zur Erhöhung der Gasausbeute einer Biogasanlage, bei dem in einer Holzvergasungsanlage durch Umwandlung von Holz Holzgas gewonnen und das Holzgas in einen Biogasfermenter eingespeist wird, in dem zunächst ein Mischgas, bestehend aus Methan, Kohlenmonoxyd und Wasserstoff und schließlich reines Methangas gebildet wird, das zur Erzeugung von Elektrizität und Wärme dient.

## Beschreibung

Die Erfindung betrifft ein Verfahren zur Erhöhung der Gasausbeute einer Biogasanlage und eine Anlage zur Durchführung des Verfahrens.

Es ist bekannt, den Festbrennstoff Holz in einer Holzvergasungsanlage in ein brennbares Gas (Pyrolysegas), das sogenannte Holzgas, umzuwandeln. Dieser Vergasungsprozeß beruht auf dem Effekt einer unvollständigen Verbrennung , bei der im wesentlichen Kohlenmonoxid und Wasserstoff entstehen. Um das so erzeugte Holzgas motorentauglich zu machen, muß es in aufwendigen Anlagen gereinigt und gekühlt werden. Dazu werden Gravitationsfilter, Zyklone, Elektrofilter, sogenannte Röhrenkühler und evtl. Gewebefilter benötigt.

Ferner ist es bekannt, in Biogasanlagen durch Umsetzung von Gülle, die in einen Biogasfermenter gepumpt und dort ausgefault wird, im wesentlichen aus Methan bestehendes , sogenanntes Biogas zu erzeugen, das in einem Blockheizkraftwerk, kurz einer BHKW-Station, in Elektrizität und Wärme umgewandelt wird.

Aufgabe der Erfindung besteht darin, die Leistungsfähigkeit der Biogasanlage zu steigern.

Nach der Erfindung wird dies dadurch erreicht, dass in einer Holzvergasungsanlage durch Umwandlung des Festbrennstoffes Holz, Holzgas gewonnen und das noch ungereinigte Holzgas in einen Biogasfermenter eingespeist wird, in dem die Verunreinigungen des Holzgases aufgeschlossen werden, und dass in dein Biogasfermenter zunächst ein Mischgas , bestehend aus Methan, Kohlenmonoxid und Wasserstoff gebildet wird , das durch weitere Fortsetzung der im Biogasfermenter ablaufenden Vorgänge schließlich zu reinem Methangas umgesetzt wird und zur Erzeugung von Elektrizität und Wärme einsetzbar ist..

Ein weiterer Gegenstand der Erfindung ist eine Anlage zur Durchführung des erfindungsgemäßen Verfahrens.

Durch die Einleitung des noch ungereinigten Holzgases in die im Biofermenter befindliche Gülle werden die Teerbestandteile durch Einwirkung der bei der Verfaulung der Gülle vorhandenen Bakterien zu Methangas umgewandelt. Staubpartikel werden an dem vorhandenen Substrat angebunden. Es entsteht_ein Mischgas, bestehend aus Methan. Durch Verlängerung der Arbeitsphase im Biofermenter entsteht schließlich reines Methangas.

Der Vorteil dieses Verfahrens liegt darin, dass einerseits die aufwendige Anlage zur Reinigung des im Holzgasreaktor gebildeten Holzgases entfallen kann, und dass andererseits die Biogasanlage mit größerem Wirkungsgrad betrieben werden kann.

Während es zunächst mit Hilfe dieses Verfahrens möglich war, die Leistung von Biogasanlagen um mindestens 50% zu steigern, hat es sich gezeigt, dass schließlich eine 100%ige Leistungssteigerung erzielt werden kann. Das heißt mit anderen Worten, die Wirtschaftlichkeit der Biogasanlage wird beachtlich verbessert..

Es ist vorteilhaft, die Leistung des Holzgasreaktors schnell von 0% auf 100 % zu steuern.. Damit ist die Biogasanlage zum ersten Mal in der Lage von der Grundlast zur Spitzenlast, bzw. zwischen 50% und 100% Leistung zu fahren. Es kann erwartet werden, das die Wirtschaftlichkeit der Anlage noch so weit verbessert werden kann, dass die Stromleistung bis zur doppelten Kapazität der bisherigen Leistung der Biogasanlage steigt..

Um dieses Verfahren durchführen zu können sind folgende Techniken vorteilhaft:
1. Ein Druckgebläse, welches das Gas mit dem notwendigen Druck fördert. Hier kann es vorteilhaft sein, ein sogenanntes Hochdruckgebläse einzusetzen.
2. Die Vermischung des Holzgases mit dem Güllesubstrat kann entweder im Biogasfermenter selbst erfolgen, man könnte aber auch die Vermischung in einem gesonderten Behälter durchführen und dann die Mischung in den Fermenter einspeisen.
3. In jedem Fall wird das Holzgas mittels einer Injektoranlage mit der Flüssigkeit zusammengebracht.
4. Diese Injektoranlage kann zum Beispiel als Eindüsungsanlage ausgebildet sein bestehend aus einer waagerechten Ringleitung, verbunden mit Lanzenrohren, an deren unterem Ende Gummidüsen angebracht sind, aus denen das Gas durch den Überdruck, der durch ein Gebläse erzeugt wird, ausströmt und in die Gülle eingeleitet wird. Wird das Gebläse abgeschaltet, schließen sich die Düsen, dann kann keine Gülle in die Lanzenrohre eindringen. Aber selbst wenn dieser Fall eintreten sollte, würde der Gasdruck die Flüssigkeit wieder nach unten wegdrücken.

Je nach Größe des Biofermenters sind 4 bis 12 Lanzendüsenanordnungen notwendig. Durch spezielle Anordnung des Gasaustrittswinkels aus den Düsen kann die Fermenterflüsssigkeit in Rotation gebracht werden. Damit wird ein weiteres mechanisches Rührwerk überflüssig.

Es hat sich gezeigt , dass das in die Fermenterflüssigkeit eingeleitete Holzgas einen zusätzlichen Aufheizeffekt des Fermentersubstrates bewirkt, das heißt, es ersetzt eine aufwendige Fermenterheizung. Darin ist ein weiterer energetischer Vorteil des erfindungsgemäßen Verfahren zu sehen.

Die Gaseindüsungsanlage besteht aus Edelstahl A 4, ebenso wie das Druckgebläse.

Vorteilhafte Ausbildungen des Verfahrens und der zu seiner Durchführung geeigneten Anlage ergeben sich aus den Unteransprüchen.

Das erfindungsgemäße Verfahren wird nachstehend anhand der Zeichnung erläutert. Hierin zeigen:

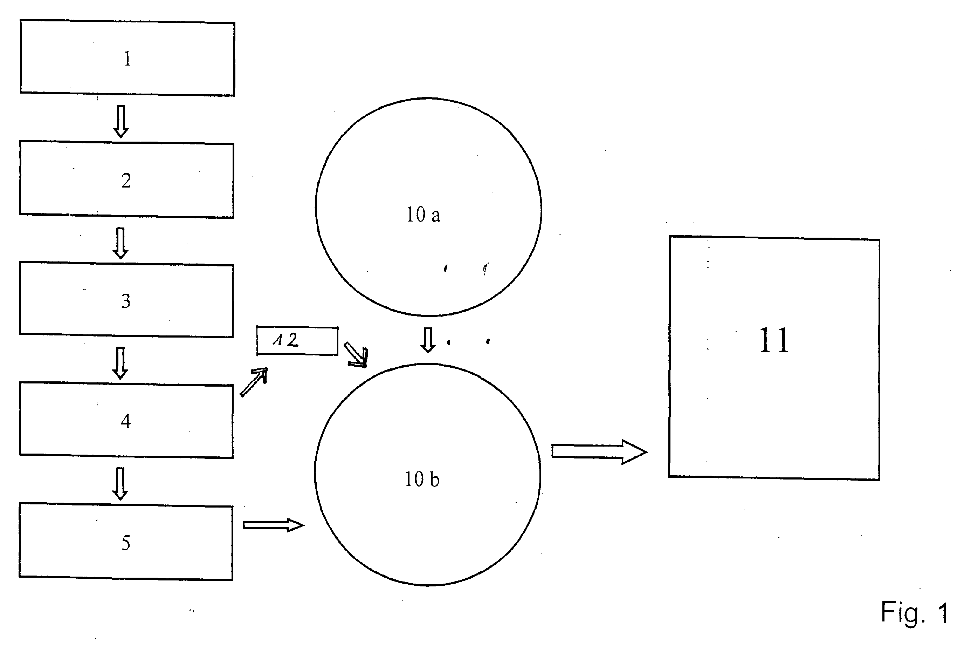

Fig. 1 ein schematisches Blockbild der Anlage,

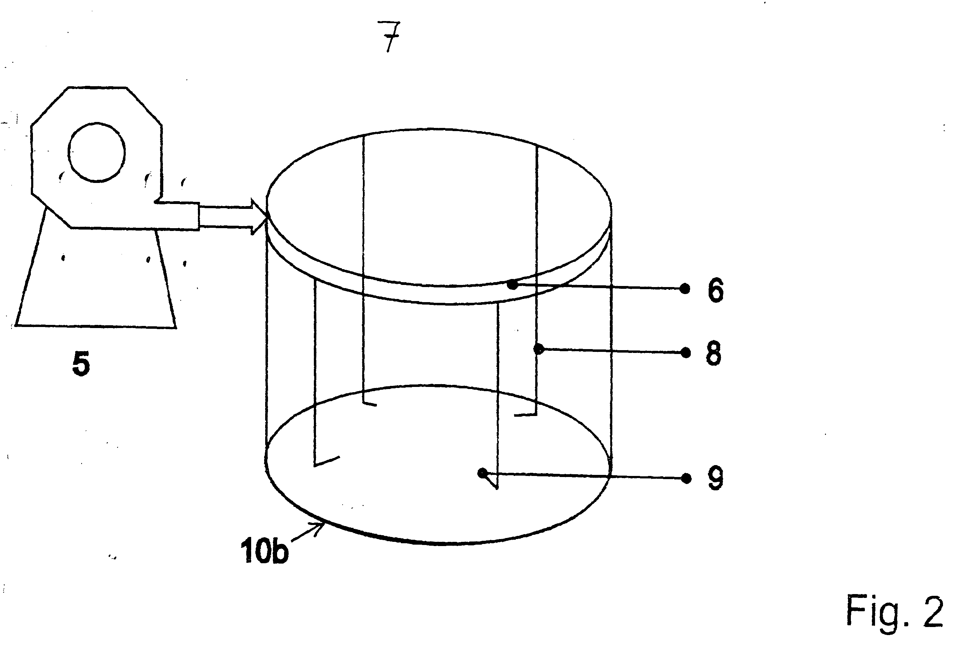

Fig. 2 eine schematische Darstellung der Eindüsungsanlage und

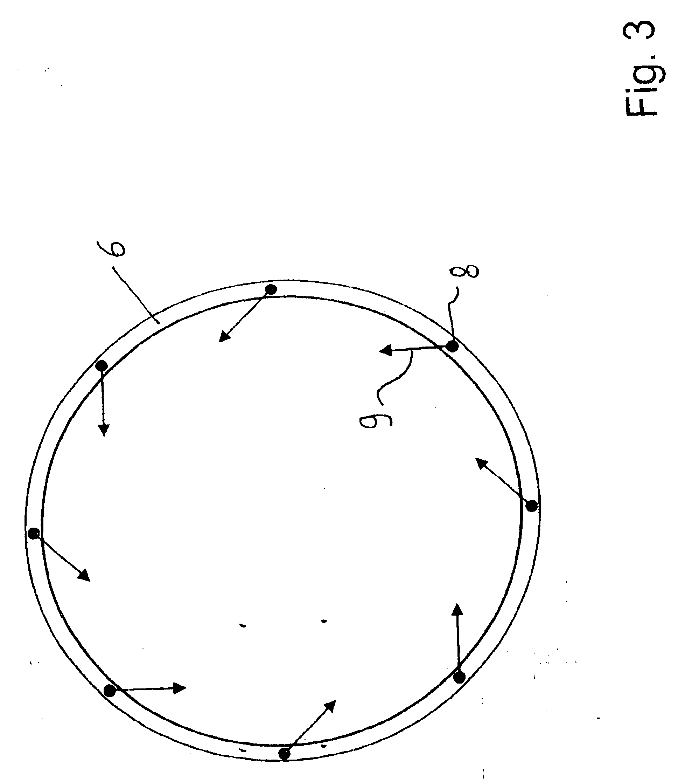

Fig. 3 eine Ansicht von unten auf die Ringleitung der Eindüsungsanlage.

Das Holz wird' in einem Häcksler 1 zu Hackschnitzeln einer Größe von etwa 40 bis 50 mm . Kantenlänge zerkleinert. In einem Holzgasreaktor 2 erfolgt bei einer Temperatur von mindestens 800°C bis maximal 1000° C die Trocknung, Entgasung und Vergasung des Holzes, wobei in der ersten Reaktionszone die Trocknung erfolgt, im zweiten Abschnitt das Holz entgast wird und die flüchtigen Bestandteile in die Gasphase übergeführt werden und in der 3. Zone der Festrückstand zu Kohlenmonoxid vergast wird.

Das gebildete Holzgas enthält noch Teere und Staubpartikel. Die groben Bestandteile werden in einem Zyklonfilter 3 abgeschieden. Anschließend wird in der Regel das Gas in i einem Wassergaskühler 4 gekühlt und dann über ein Druckgebläse 5 in einen Biogasfermenter 10a,b überführt. Der Biogasfermenter 10ab besteht in dem beschriebenen Ausführungsbeispiel aus aus zwei hintereinandergeschalteten Biogasfermentern 10a und 10b In diesem Fall wird das Gas von einem Hochdruckgebläse 5 in eine oberhalb des Biogasfermenters 10b befindliche Ringleitung 6 einer Eindüsungsanlage 7 gedrückt. Man könnte aber auch nur einen einzigen Biogasfermenter verwenden.

Der im Wassergaskühler 4 abgeschiedene Sumpf, der hauptsächlich aus Staub besteht , wird mit einer in einem Mischbehälter befindlichen Pumpe 12 ebenfalls in den Biogasfermenter 10b gepumpt. Der bei der Pyrolyse im Holzgasreaktor 2 gebildete Teer ist in dem gekühlten Gas enthalten und wird mit diesem über die Eindüsungsanlage 7in den Biogasfermenter 10b übergeführt.

Die Eindüsungsanlage 7 umfaßt das Hochdruckgebläse 5, die Ringleitung 6 und ein Lanzendüsensystem 8. Der Auslaß der Düsen 9 des Lanzendüsensystems 8 befindet sich ca. 2 bis 4 m unterhalb des Flüssigkeitsspiegels der in dem Biogasfermenter 10b befindlichen Gülle. Die Düsen 9 sind in einem Winkel zwischen 45° und 60° bezogen auf die Wandung des Biogasfermenters 10b ausgerichtet, um die Fermenterflüssigkeit durch das austretende Gas in Rotation zu versetzen.
Das Lanzendüsensystem 8 sorgt für eine feine Verteilung des eingeleiteten Gases im Biogasfermenter 10b. Das im Biogasfermenter 10b zunächst gebildete Gasgemisch besteht aus Methan, Kohlenmonoxid und Wasserstoff, dieses wird aber durch Fortsetzung des eingeleiteten Verfahrensablaufs zu Methan umgesetzt , das zum Beispiel dann einem Blockkraftheizwerk 11 zur Erzeugung der Elektrizität und Wärme zugeführt werden kann..

## Patentansprüche

1. Verfahren zur Erhöhung der Gasausbeute einer Biogasanlage, **dadurch ge- kennzeichnet**, dass in einer Holzvergasungsanlage durch Umwandlung des Festbrennstoffes Holz Holzgas gewonnen und das noch ungereinigte Holzgas in einen Biogasfermenter eingespeist wird in dem die Verunreinigungen des Holzgases aufgeschlossen werden, und dass in dem Biogasfermenter zunächst ein Mischgas, bestehend aus Methan, Kohlenmonoxyd und Wasserstoff gebildet wird, das durch Fortsetzung der im Biogasfermenter ablaufenden Vorgänge schließlich zu reinem Methangas umgesetzt und zur Erzeugung von Elektrizität und Wärme eingesetzt wird.

2. Verfahren nach Anspruch 1, **dadurch gekennzeichnet, dass** die Verunreinigungen des Holzgases Teer und Staubpartikel enthalten, die durch im Biogasfermenter vorhandene Bakterien zu Methan umgewandelt werden, und dass die Staubpartikel an dem bestehenden Substrat gebunden werden.

3. Verfahren nach Anspruch 1 oder 2, **dadurch gekennzeichnet, dass** die Einleitung des ungereinigten Holzgases in den Biogasfermenter mit hohem Druck über ein Lanzendüsensystem erfolgt.

4. Verfahren nach einem der Ansprüche 1 bis 3, **dadurch gekennzeichnet, dass** die Einleitung des Holzgases zwischen etwa 2 und 4 m unter dem Flüssigkeitsspiegel der Gülle im Biogasfermenter erfolgt.

5. Anlage für die Durchführung des Verfahrens nach einem der Ansprüche 1 bis 4 , **dadurch gekennzeichnet, dass** eine Holzvergasungsanlage (2) für die Umwandlung des Festbrennstoffes Holz in ungereinigtes Holzgas über eine Zuführungsvorrichtung (5, 7) für das Holzgas mit einem Biogasfermenter (10b) verbunden ist, dem das Holzgas zugeführt wird, und dass der Biogasfermenter (10b) mit einem Blockheizkraftwerk (BHKW) (11) verbunden ist, dem das im Biogasfermenter (10a,b) gebildete Biogas zusammen mit dem nach Umsetzing seiner Verunreinigungen gebildeten Holzgas als Mischgas zugeführt wird.

6. Anlage nach Anspruch 5 , **dadurch gekennzeichnet, dass** die Zuführungsvorrichtung (5,7) als eine Eindüsungsanlage (7) ausgebildet ist, die ein Hochdruckgebläse (5), eine Ringleitung (6) und ein Lanzendüsensystem (8) mit Austrittsdüsen (9) umfasst.

7. Anlage nach einem der Ansprüche 5 oder 6, **dadurch gekennzeichnet, dass** die Austrittsdüsen (9) der Eindüsungsanlage (7) etwa 2 bis 4 m unter dem Flüssigkeitsspiegel der in dem Biogasfermenter (10b) befindlichen Gülle, innerhalb der Wandung des Biogasfermenters (10b) angeordnet sind..

8. Anlage nach einem der Ansprüche 5 bis 7, **dadurch** gekennzetchnet, dass die Austrittsdüsen (9) in einem Winkel von 45° bis 60° , bezogen auf die Wandung des Biogasfermenters (10b) ausgerichtet sind.

9. Anlage nach einem der Ansprüche 5 bis 8, **dadurch gekennzeichnet, dass** zwischen dem Holzgasreaktor (2) und der Zuführungsvorrichtung (5,7) ein Zyklonfilter(3) und diesem nachgeschaltet ein Wassergaskühler(4) vorgesehen sind.
